# EUROPEAN PATENT APPLICATION

(11) **EP 3 745 119 A1**
(43) Date of publication of application: **02.12.2020**
(21) Application number: 18902498.7
(22) Date of filing: 26.11.2018
(51) Int. Cl.: G01N 23/04, G01N 23/18

(54) **INSPECTION LINE**

(30) Priority: 26.01.2018 JP 2018011294
(71) Applicant: System Square Inc., Nagaoka-shi Niigata 9402121 (JP)
(72) Inventor: HASEGAWA Isao, Nagaoka-shi,Niigata 940-2121 (JP); KANNO Atsushi, Nagaoka-shi,Niigata 940-2121 (JP); HATAKEYAMA Takuya, Nagaoka-shi,Niigata 940-2121 (JP)
(74) Representative: Müller-Boré & Partner Patentanwälte PartG mbB
(86) International application number: PCT/JP2018/043336
(87) International publication number: WO 2019/146235

(57) **Abstract**

An inspection line including an inspection apparatus capable of inspecting an inspection target by simple structure and control are provided.

An X-ray inspection apparatus including: an irradiation unit for irradiating X-rays to an inspection target in an inspection region a detection unit for detecting an intensity distribution of X-rays transmitted through the inspection target; a control unit for generating an X-ray transmission image of the inspection target based on the intensity distribution of X-rays detected by the detection unit and determining acceptance/rejection of the inspection target; and a conveying unit for causing the inspection target to pass through the inspection region, sorting the inspection target into the passed inspection target and the rejected inspection target according to a result of acceptance/rejection determination by the control unit, and conveying the rejected inspection target to a workbench for performing work on the inspection target without passing through the inspection region.

## Description

### TECHNICAL FIELD

The present invention relates to an inspection line provided with an inspection apparatus for inspecting an inspection target by irradiating the inspection target with a detection wave.

### BACKGROUND ART

An inspection apparatus is known in which an irradiation unit for irradiating a detection wave and a detection unit for detecting the detection wave are opposed to each other, an inspection target is sequentially conveyed by a conveying device such as a conveyor so as to pass therebetween, an image of the inspection target is constructed on the basis of an intensity distribution of the detection wave obtained when the inspection target passes between the irradiation unit and the detection unit, and the inspection target is inspected in a non-destructive and non-contact manner using the image.

As such an inspection apparatus, an X-ray inspection apparatus using X-rays as the detection wave is known. As an application of the X-ray inspection apparatus, there is a case where food such as fish or meat is used as the inspection target, and the X-ray inspection apparatus inspects whether the foreign matter is mixed or not in the inspection target, whether the inspection target is cracked or chipped or not, and the like.

Patent Document 1 discloses an X-ray inspection apparatus capable of performing an inspection for confirming whether or not the inspection target has a foreign matter (hereinafter, referred to as a normal inspection) and an inspection for reconfirming the inspection target (on which corrective work such as removing foreign objects is performed as necessary) in which the foreign matter is found by the normal inspection (hereinafter referred to as a re-inspection) with one X-ray inspection apparatus. In this X-ray inspection apparatus, a conveyor for the normal inspection that conveys in the forward direction and a conveyor for the re-inspection that conveys in the reverse direction are arranged side by side and passed through the X-ray inspection apparatus.

### PRIOR ART REFERENCES

### PATENT DOCUMENTS

Patent Document 1: Japanese Unexamined Patent Publication 2014-048178

### SUMMARY OF THE INVENTION

### PROBLEMS TO BE SOLVED BY THE INVENTION

However, Patent Document 1 has a problem in that control for conveyance and imaging becomes complicated because normal inspection and re-inspection of an inspection target are performed by arranging conveyors having different conveyance directions in one inspection apparatus. In addition, since conveyors having different conveying directions are arranged side by side and passed through the X-ray inspection apparatus, the Patent Document has another problem that it is difficult to inspect an inspection target having a wide width.

The present invention has been made in view of the above problems, and an object thereof is to provide an inspection line including an inspection apparatus capable of inspecting an inspection target by a simple structure and control.

### MEANS FOR SOLVING THE PROBLEMS

In order to solve the above-mentioned problems, an inspection line according to the present invention comprises: an X-ray inspection apparatus including: an irradiation unit for irradiating X-rays to an inspection target in an inspection region; a detection unit for detecting an intensity distribution of X-rays transmitted through the inspection target; and a control unit for generating an X-ray transmission image of the inspection target based on the intensity distribution of X-rays detected by the detection unit and determining acceptance or rejection of the inspection target; and a conveying unit for causing the inspection target to pass through the inspection region, sorting the inspection targets into acceptable inspection targets and rejected inspection targets according to a result of acceptance/rejection determination by the control unit, and conveying the rejected inspection target to a workbench for performing work on the inspection target without passing through the inspection region.

According to such a configuration, the structure and control can be simplified. In addition, a wide inspection target can be inspected by effectively utilizing the width of the inspection region.

The inspection line of the present invention may further include a display unit disposed at a position visually recognizable by a worker on the workbench, and an operation unit for receiving a predetermined operation. The X-ray inspection apparatus may include a storage unit that sequentially stores the X-ray transmission images generated by the control unit in association with the determination result of acceptance or rejection and information indicating the inspection order. The control unit may cause the display unit to display the X-ray transmission image having the oldest inspection order among the X-ray transmission images with which the determination result of "rejected" is associated and not yet displayed on the display unit in response to the operation unit receiving the predetermined operation. With this configuration, the control unit can cause the display unit to display the X-ray transmission images of the rejected inspection target in the order of inspection in response to receiving the predetermined operation from the operation unit.

In the present invention, the conveying unit may include a waiting unit that temporarily holds the sorted rejected inspection target in front of the workbench. With this configuration, it is possible to temporarily hold the rejected inspection target while the worker who performs corrective work is performing the work.

In the present invention, it is preferable to further include a sensor that detects there is no room to accept the rejected inspection target in the waiting unit, and the control unit may perform control so as to stop conveyance by the conveying unit when the sensor detects that there is no room to accept the rejected inspection target. With this configuration, the sensor can detect that the rejected inspection target is filled up to the entrance of the waiting unit, and based on that, the control unit can control to stop the conveying unit.

In the present invention, the waiting unit may be configured so that a plurality of the inspection targets may be allowed to wait in the waiting unit, and the inspection targets waiting in the waiting unit may be moved to the workbench in the order in which they are conveyed to the waiting unit. With this configuration, the worker on the workbench can perform the corrective work on the rejected inspection target corresponding to the X-ray transmission image displayed on the display unit.

In the present invention, it is preferable that the waiting unit has an inclined surface that slopes downwardly toward the workbench, and the inclined surface can hold a plurality of inspection targets. With this configuration, the waiting unit can hold a larger number of rejected inspection targets, and can sequentially move the inspection targets to the workbench in a first-in-first-out manner without providing a drive mechanism in the waiting unit.

In the inspection apparatus of the present invention, it is preferable that the workbench is provided adjacent to the supply port to the inspection region. With this configuration, the worker on the workbench can put the inspection target after the corrective work into the inspection region for re-inspection without greatly moving the inspection target.

### BRIEF DESCRIPTION OF THE DRAWINGS

Fig. 1 is a perspective view showing an appearance of the inspection line.
Fig. 2 is a perspective view showing the inspection targets mounted on a tray.
Fig. 3 is a diagram showing a configuration of the X-ray inspection apparatus.
Fig. 4 is a perspective view showing an appearance of a modification of the inspection line.

### DESCRIPTION OF EMBODIMENTS

### [First embodiment]

Embodiments of the present invention will be described below with reference to the drawings. In the following description, portions already described are denoted by the same reference numerals, and the description thereof is omitted.

### [Configuration of the Inspection Apparatus]

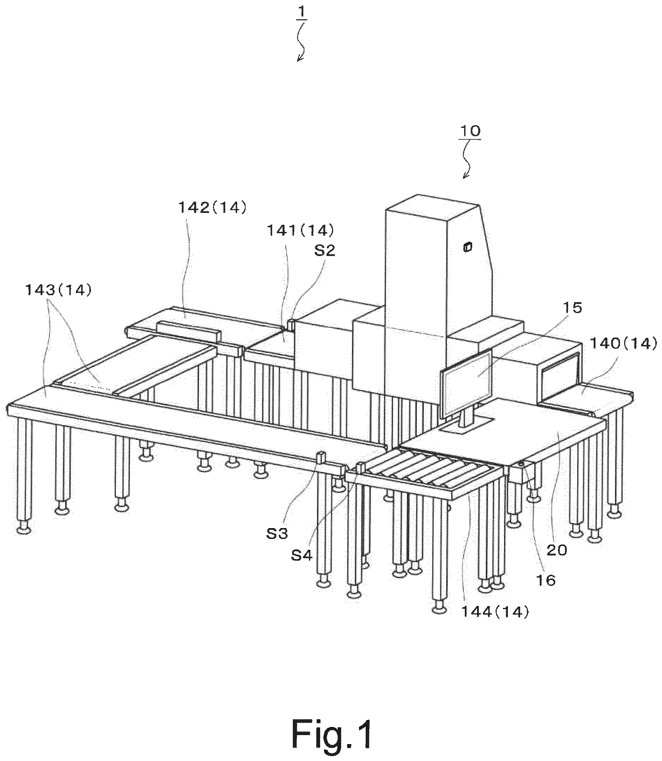

Fig. 1 is a schematic diagram illustrating an inspection line having an X-ray inspection apparatus according to the present embodiment.

The inspection line 1 is a line for performing foreign matter removal processing and inspection on an object to be processed and inspected (hereinafter referred to as inspection target W) such as fish and chicken. The inspection line 1 includes an X-ray inspection apparatus 10 that captures an X-ray transmission image of the inspection target W and determines the acceptance/rejection of the inspection target W based on the captured X-ray transmission image, and a conveying unit that conveys the inspection target W from an upstream process to pass through the X-ray inspection apparatus 10 and conveys the passed inspection target W to either a downstream process or a workbench at which corrective work is performed in accordance with the acceptance/rejection determination result. The X-ray inspection apparatus 10 may determine the presence or absence of foreign matter such as a fish pin bone (small fishbone) or a chicken residual bone based on the X-ray transmission image, and determine the inspection target W in which the foreign matter remains as "rejected".

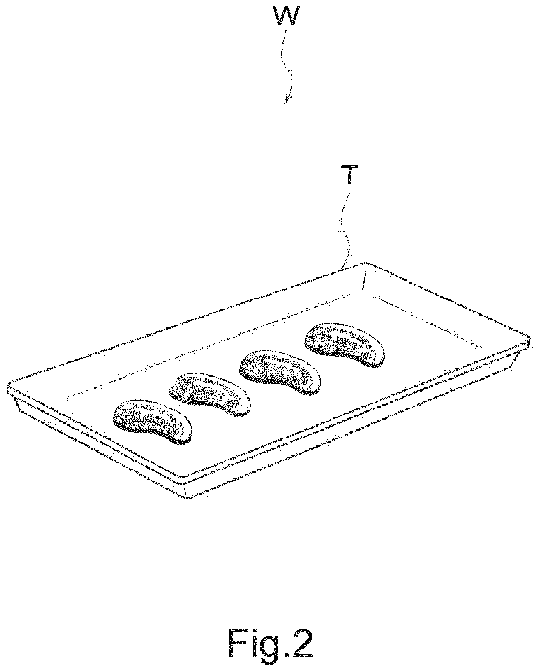

In the present embodiment, the X-ray transmission image of the inspection target W is captured by the X-ray inspection apparatus 10 in a state where the inspection target W is placed on a predetermined tray T. Fig. 2 shows an example of the tray T together with the inspection targets. The tray T is, for example, a rectangular dish-shaped container having a width of 25cm and a length of 80cm, and is formed of a material transparent to X-rays, for example, a plastic such as polyethylene or polystyrene. The tray T is supplied to the X-ray inspection apparatus 10 so that the longitudinal direction thereof is the conveying direction. As shown in Fig. 2, a plurality of inspection target may be placed on the tray T. Hereinafter, the entire inspection target (maybe a plurality thereof) placed on the tray T is collectively referred to as the inspection target W.

As shown in Fig. 1, the inspection line 1 includes the X-ray inspection apparatus 10, a conveying unit 14, a display unit 15, an operation unit 16, sensors S (S1 to S4), and a workbench 20.

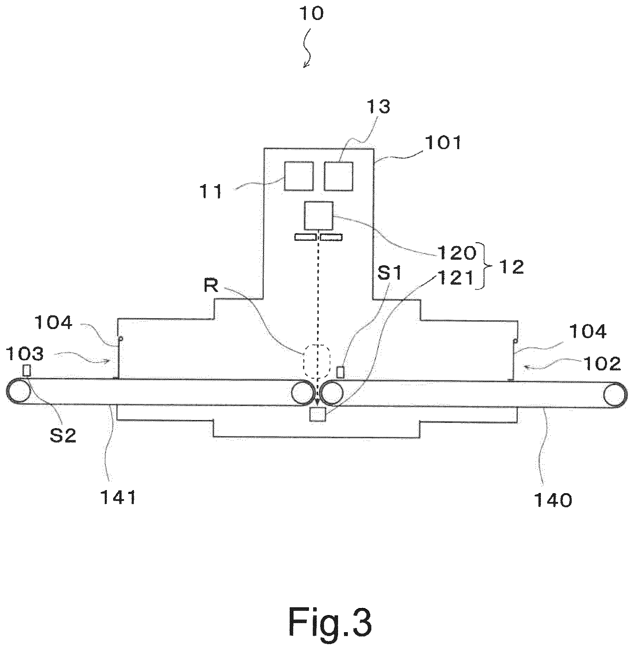

Fig. 3 is a diagram showing an internal configuration of the X-ray inspection apparatus 10. The X-ray inspection apparatus 10 includes a main body cover 101, a control unit 11, an inspection unit 12, and a storage unit 13. The main body cover 101 is a housing of the X-ray inspection apparatus 10, and functions as a shield for preventing X-rays from leaking to the outside of the X-ray inspection apparatus 10. The main body cover 101 is provided so as to cover the control unit 11, the inspection unit 12, and the storage unit 13. The main body cover 101 may further cover a part of the conveying unit 14. The main body cover 101 is provided with a supply port 102 for supplying the inspection target W to the inside and a discharge port 103 for discharging the inspection target W to the outside. The inspection target W is conveyed on the conveying surface from the supply port 102 toward the discharge port 103 by the conveying unit 14. The supply port 102 and the discharge port 103 are provided with an X-ray shield 104 which is a plate-shaped body made of metal (for example, stainless steel) capable of shielding X-rays. The X-ray shield 104 is pivotally supported so as to be rotatable by a predetermined angle toward the conveying direction so as not to hinder the movement of the inspection target W when the X-ray shield is pushed and moved by the movement of the inspection target W conveyed by the conveying unit 14. The X-ray shield 104 is normally in a closed state in which the X-ray shield 104 hangs down toward the conveying unit 14 by its own weight, and is pushed up and rotated to an open state only when the inspection target W passes under the X-ray shield 104. Therefore, a drive mechanism for driving the X-ray shield 104 to open and close is not required, and the cost can be suppressed.

The control unit 11 is constituted of a processing device such as a CPU. The control unit 11 is connected with components including the inspection unit 12, the storage unit 13, the conveying unit 14, the display unit 15, the operation unit 16, and the sensors S. The control unit 11 performs processing based on signals from the inspection unit 12, the operation unit 16, the sensor S, etc., and controls each unit in the inspection line 1. The operation of the inspection line 1 performed under the control of the control unit 11 will be described in detail later.

The inspection unit 12 includes an irradiation unit 120 for irradiating the inspection region R with X-rays, and a detection unit 121 for detecting the intensity distribution of X-rays that have transmitted through the inspection target.

The irradiation unit 120 is disposed above the center of the X-ray inspection apparatus 10. The irradiation unit 120 irradiates the detection unit 121 arranged inside the belt of the conveying unit 14 with X-rays. X-rays irradiated from the irradiation unit 120 passes through the slit and spread from the irradiation unit 120 in a substantially fan shape. Incidentally, the X-rays radiated from the irradiation unit 120 spread on a plane that is substantially perpendicular to the conveying surface of the conveying unit 14 and spread in the width direction of the conveying surface of the conveying portion 14.

Of the substantially fan-shaped region through which the X-rays radiated from the irradiation unit 120 passes, the region from the upper surface of the conveyor belt to a predetermined height is the inspection region R of the X-ray inspection apparatus 10. The X-ray inspection apparatus 10 radiates X-rays to the inspection target W passing through the inspection region R to perform an inspection.

The detection unit 121 is constituted of a line sensor in which a large number of X-ray detection elements are arranged in a line, and detects the X-rays transmitted through the inspection target W. The detection unit 121 is arranged such that the X-ray detection elements that form the line sensor are arranged in a direction substantially perpendicular to the conveying direction and substantially parallel to the conveyance surface (that is, the width direction of the conveyor belt). The detection unit 121 may detect X-rays in the gap between conveyors constituting the conveying unit 14 as shown in Fig. 3. With this configuration, the X-rays can be detected without passing through the conveyor belt, so that the signal-to-noise ratio can be improved. Each X-ray detection element detects an X-ray at each position on the line, and outputs the intensity of the X-ray (transmitted X-ray dosage) detected at each position to the control unit 11. The control unit 11 generates the X-ray transmission image of the inspection target based on the intensity distribution of X-rays detected by the detection unit 121.

The storage unit 13 is constituted of a storage device such as a RAM or a hard disk. The storage unit 13 stores various programs to be executed by the control unit. The storage unit 13 stores the X-ray transmission image formed by the control unit 11 based on the output of the detection unit 121. The storage unit 13 sequentially stores the X-ray transmission images in association with the determination result of acceptance/rejection of the inspection target shown in the image and information indicating the inspection order.

The conveying unit 14 conveys the inspection target W to the X-ray inspection apparatus 10, the downstream process, the workbench 20, and the like. The term "conveying to the workbench 20" includes not only conveying the inspection target W onto the workbench 20 but also conveying to the vicinity of the workbench 20 (for example, a position that can be reached by the worker who works on the workbench 20). The conveying unit 14 includes a loading conveyor unit 140, a discharging conveyor unit 141, a sorting unit 142, a return conveyor unit 143, and a waiting unit 144.

Further, each part of the conveying unit 14, a sensor S (S1 to S4) is provided. The sensor S detects whether or not there is the inspection target W at a position where the sensor S is provided. The output of the sensor S is connected to the control unit 11, and the presence or absence of the inspection target W is transmitted to the control unit 11. As the sensor S, for example, a regression reflecting photoelectric sensor can be used.

The loading conveyor unit 140 conveys the inspection target W from the outside to the inspection region R in the X-ray inspection apparatus 10. As the loading conveyor unit 140, for example, a belt conveyor can be used, and the inspection target W is conveyed by driving a motor to rotate the roller. A sensor S1 is provided between the supply port 102 to the X-ray inspection apparatus 10 and the inspection region R on the conveying path of the loading conveyor unit 140. The sensor S1 detects that the inspection target W conveyed by the loading conveyor unit 140 reaches the inspection region R soon. Triggered by the detection of the arrival of the inspection target W by the sensor S1, the control unit 11 generates the X-ray transmission image of the inspection target W based on the intensity distribution of X-rays detected by the detection unit 12, and stores the X-ray transmission image in the storage unit 13.

The discharging conveyor unit 141 discharges the inspection target W from the inspection region R to the outside of the X-ray inspection apparatus 10. As the discharging conveyor unit 141, similarly to the loading conveyor unit 140, for example, a belt conveyor can be used. A sensor S2 is provided between the discharge port and the sorting unit 142 on the conveying path of the discharging conveyor unit 141. The sensor S2 detects that the inspection target W conveyed by the discharging conveyor unit 141 reaches the sorting unit 142 soon.

The sorting unit 142 sorts the inspection target W into the acceptable product and the rejected product in accordance with the result of the acceptance/rejection determination of the inspection target W and the output of the sensor S2. The sorting unit 142 sorts the inspection target W so as to proceed to the downstream process for the accepted products and to the return conveyor unit 143 for the rejected products. As the sorting unit 142, a flipper type sorting machine provided with a distributing arm on a belt conveyor, or a conveyor capable of conveying in a plurality of directions by a combination of a belt conveyor and a roller conveyor can be used.

The return conveyor unit 143 conveys the inspection target W sorted as a rejected product by the sorting unit 142 to the waiting unit 144 without passing through the inspection region R. Since the return conveyor unit 143 does not pass through the inspection region R, the direction in which the inspection target W is conveyed from the outside to the inspection region R is only one direction from upstream to downstream. As a result, even for the inspection target W having a wide width, the X-ray transmission image can be acquired by effectively utilizing the width of the inspection unit 12. Further, it is possible to avoid complicated control for acquiring the X-ray transmission image. As the return conveyor unit 143, similarly to the loading conveyor unit 140, for example, a belt conveyor can be used.

A sensor S3 is provided in the vicinity of the downstream end of the return conveyor unit 143 on the conveyance path (that is, near the boundary with the waiting unit 144). The sensor S3 detects that the inspection target W is present immediately before the waiting unit 144.

The waiting unit 144 functions as a region where the rejected product conveyed from the return conveyor unit 143 is temporarily held in front of the workbench. The waiting unit 144 is provided between the workbench 20 and the downstream end portion of the return conveyor unit 143. As the waiting unit 144, a roller conveyor to which no driving force is applied can be used. Alternatively, a part of the workbench 20 may be the waiting unit 144. The worker who works on the workbench 20 takes out one of the inspection targets W held in the waiting unit 144 in the order in which they are conveyed to the waiting unit 144 (that is, in a first-in-first-out manner), moves the one to the workbench 20, and performs work on the inspection target W. In addition, a sensor S4 is provided in the vicinity of the entrance of the waiting unit 144 (that is, near the boundary with the return conveyor unit 143). The sensor S4 detects that the inspection target W is present up to the rear end of the waiting unit 144 (that is, the waiting unit 144 has no room to accept another inspection target W).

The display unit 15 is a display that displays an X-ray transmission image of a rejected product, and is disposed at a position that can be visually recognized by a worker working on the workbench 20. For example, the display unit 15 may be provided at a position facing the worker. Thereby, the worker can remove the foreign matter contained in the rejected product while visually observing the display unit 15.

The operation unit 16 receives an input inputted by the worker in charge of removing a foreign matter from rejected products, and transmits the input to the control unit 11. The operation unit 16 may be, for example, a push button switch, and may be provided at a position where a worker working on the workbench 20 can operate without moving.

The workbench 20 is a table for the worker to perform corrective work on the rejected product, and is provided between the waiting unit 144 and the supply port 102. The corrective work is, for example, a work of removing foreign matter remaining in the inspection target W. On the workbench 20, the worker takes out the inspection target W in the order in which it arrives the waiting unit 144 and performs the corrective work.

### [Explanation of X-ray inspection]

Next, the flow of the X-ray inspection of the inspection target W performed on the inspection line 1 will be described together with the control performed by the control unit 11. It should be noted that in the following description, the operation in which the subject is not specified is performed by the control unit 11.

The X-ray inspection in the inspection line 1 is performed on the inspection target W conveyed by the loading conveyor unit 140. When the loading conveyor unit 140 conveys the inspection target W to the installation position of the sensor S1, the control unit 11 controls the inspection unit 12 so as to detect the intensity distribution of the transmitted X-rays of the inspection target W passing through the inspection region R in response to the inspection target W reaching the sensor S1. The control unit 11 generates the X-ray transmission image of the inspection target W based on the detected intensity distribution of X-rays.

Subsequently, the control unit 11 determines acceptance/rejection of the inspection target W based on the generated X-ray transmission image of the inspection target. Then, the control unit 11 sequentially stores the X-ray transmission image of the inspection target W in the storage unit 13 in association with the determination result of acceptance/rejection and the information indicating the inspection order.

Subsequently, the control unit 11 controls the discharging conveyor unit 141 to send out the inspection target W from the inspection region R to the outside. When the inspection target W reaches the installation position of the sensor S2, the control unit 11 controls the sorting unit 142 so as to sort the accepted product to the downstream process and the rejected product to the return conveyor unit 143 in accordance with the determination result of acceptance/rejection of the inspection target W.

The inspection target W sorted to the return conveyor unit 143 by the sorting unit 142 is conveyed to the waiting unit 144 by the return conveyor unit 143. The inspection target W that has reached the waiting unit 144 is temporarily held by the waiting unit 144 until the worker moves the inspection target W to the workbench 20.

Here, the control unit 11 controls the conveying unit 14 based on the detection results of the sensors S3 and S4 so that the inspection target W does not overflow from the waiting unit 144. That is, when both the sensor S3 and the sensor S4 detect the presence of the inspection target W (i.e., when there is no room to accept the inspection target W in the waiting unit 144 and the inspection target W is located at the downstream end portion of the return conveyor unit 143), the control unit 11 stops the conveyance by the return conveyor unit 143. Thereby, it is possible to prevent the inspection target W from overflowing from the waiting unit 144. After that, when the sensor S3 or the sensor S4 stops detecting the inspection target W as a consequence of the worker's moving operation of the inspection target W from the waiting unit 144 to the workbench 20 or removal of the inspection target W at the downstream end portion of the return conveyor unit 143, the control unit 11 controls to restart the conveyance by the return conveyor unit 143.

In this manner, the worker moves the inspected targets W that has reached the waiting unit 144 and is held therein to the workbench 20 in the order in which they arrived at the waiting unit 144 (that is, in a first-in-first-out manner) to perform the corrective work.

At this time, the worker moves the inspection target W from the waiting unit 144 and operates the operation unit 16. When the operation unit 16 receives a predetermined operation, the control unit 11 causes the display unit 15 to display an image corresponding to the inspection target W moved from the waiting unit 144 among the X-ray transmission images stored in the storage unit 13. Specifically, the control unit 11 controls the display unit 15 to display the X-ray transmission image that is associated with the rejection determination result and is not yet displayed on the display unit 15 and has the oldest inspection order. Whether or not the image has already been displayed on the display unit 15 may be considered to have been displayed if the inspection order is older than that of the X-ray transmission image displayed on the display unit 15 at that time. Alternatively, information indicating whether or not the image has already been displayed on the display unit 15 may be stored in association with the X-ray transmission image. Then, when displaying the X-ray transmission image on the display unit 15, it may be determined whether the X-ray transmission image has already been displayed or not based on the information indicating whether the X-ray transmission image has already been displayed or not, and this information may be updated when displaying an image that has not yet been displayed. With this configuration, the X-ray transmission image corresponding to the inspection target W on which the worker performs the corrective work on the workbench 20 can be displayed on the display unit 15. Then, the worker performs a corrective work on the inspection target W while checking the X-ray transmission image displayed on the display unit 15.

When the worker finishes the corrective work on the inspection target W, the worker moves the inspection target W after the corrective work to the loading conveyor unit 140 in order to perform the re-inspection by the X-ray inspection apparatus 10. Then, the worker moves the next inspection target W from the waiting unit 144 to the workbench 20, and operates the operation unit 16 to switch the image to be displayed on the display unit 15. The X-ray transmission image displayed on the display unit 15 is an X-ray transmission image corresponding to the inspection target W newly taken out from the waiting unit 144.

With the configuration and operation described above, the inspection line 1 can inspect the inspection target W by a simple structure and control as described above. In addition, since the return conveyor unit 143 does not pass through the X-ray inspection apparatus 10, it is possible to inspect a wide inspection target W by effectively utilizing the width of the inspection region R. In addition, since the X-ray transmission image of the inspection target W displayed on the display unit 15 and the inspection target W to be corrected on the workbench correspond to each other, the worker can efficiently perform the corrective work while checking the X-ray transmission image displayed on the display unit 15.

### [Modification of embodiment]

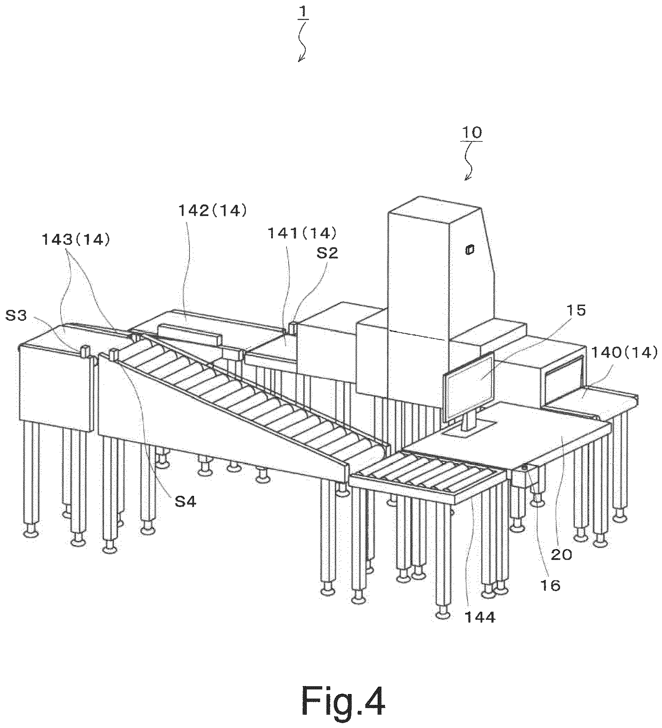

Although the present embodiment has been described above, the present invention is not limited to these examples. For example, as shown in Fig. 4, for the waiting unit 144, it may be provided with an inclined surface to be downhill toward the workbench 20 so that the path from the vicinity of the outlet of the return conveyor unit 143 to the workbench 20 is downhill. Thereby, the waiting unit 144 can hold a plurality of inspection targets on the inclined surface, and can hold more inspection target W.

In the above embodiment, the inspection line including the X-ray inspection apparatus using X-rays as the detection wave has been described as an example, but the inspection line may be provided with an inspection apparatus that uses detection wave other than X-rays such as γ rays, ultraviolet rays, visible rays, infrared rays, and terahertz waves.

Further, an invention in which a person skilled in the art appropriately add, delete, or change the design of the above-described embodiments, or a combination of the features of the respective embodiments as appropriate is also included in the scope of the present invention as long as it has the gist of the present invention.

### Reference Signs List

- 1: Inspection Line
- 10: X-ray Inspection Apparatus
- 101: Main Body Cover
- 11: Control Unit
- 12: Inspection Unit
- 120: Irradiation Unit
- 121: Detection Unit
- 13: Storage Unit
- 14: Conveying Unit
- 140: Loading conveyor unit
- 141: Discharging conveyor unit
- 142: Sorting unit
- 143: Return Conveying Unit
- 144: Waiting Unit
- 15: Display Unit
- 16: Operation Unit
- S1, S2, S3, S4: Sensor
- 20: Workbench
- W: Inspection Target

## Claims

1. An inspection line comprising:
an X-ray inspection apparatus including:
an irradiation unit for irradiating X-rays to an inspection target in an inspection region;
a detection unit for detecting an intensity distribution of X-rays transmitted through the inspection target; and
a control unit for generating an X-ray transmission image of the inspection target based on the intensity distribution of X-rays detected by the detection unit and determining acceptance or rejection of the inspection target; and
a conveying unit for causing the inspection target to pass through the inspection region, sorting the inspection target into the acceptable inspection target and the rejected inspection target according to a result of acceptance/rejection determination by the control unit, and conveying the rejected inspection target to a workbench for performing work on the inspection target without passing through the inspection region.

2. The inspection line according to claim 1, further comprising:
a display unit disposed at a position visually recognizable by a worker on a workbench; and
an operation unit for receiving a predetermined operation,
wherein,
the X-ray inspection apparatus includes a storage unit that sequentially stores the X-ray transmission images generated by the control unit in association with the determination result of acceptance/rejection and information indicating the inspection order, and
the control unit causes the display unit to display the X-ray transmission image having the oldest inspection order among the X-ray transmission images in which the determination result of "rejected" is associated and not yet displayed on the display unit in response to the operation unit receiving the predetermined operation.

3. The inspection line according to claim 1 or 2, wherein the conveying unit includes a waiting unit that temporarily holds the sorted rejected inspection target in front of the workbench.

4. The inspection line according to claim 3, further comprising a sensor that detects there is no room to accept the rejected inspection target in the waiting unit,
wherein the control unit performs control so as to stop conveyance by the conveying unit when the sensor detects that there is no room to accept the rejected inspection target.

5. The inspection line according to claim 3 or 4, wherein the waiting unit is configured so that a plurality of the inspection targets are allowed to wait in the waiting unit, and
the inspection targets waiting in the waiting unit are moved to the workbench in the order in which the inspection targets are conveyed to the waiting unit.

6. The inspection line according to any one of claims 3 to 5, wherein the waiting unit has an inclined surface that slopes downwardly toward the workbench, and the inclined surface can hold a plurality of inspection targets.

7. The inspection line according to any one of claims 1 to 6, wherein the workbench is provided adjacent to a supply port to the inspection region.
